# EUROPÄISCHE PATENTSCHRIFT

(11) **EP 3 930 563 B1**
(45) Veröffentlichungstag und Bekanntmachung des Hinweises auf die Patenterteilung: **22.04.2026**
(21) Anmeldenummer: 19808968.2
(22) Anmeldetag: 28.10.2019
(51) Int. Cl.: A61B 5/00, G06T 7/00, G06V 10/44, G06V 10/764, G06V 10/82, G06V 40/10, G06N 3/0464, G06N 3/09, G06N 3/096

(54) **VORRICHTUNG ZUR AUSFÜHRUNG EINES VERFAHRENS ZUR EVALUIERUNG VON HAUTLÄSIONEN UNTER VERWENDUNG KÜNSTLICHER INTELLIGENZ**
DEVICE FOR PERFORMING A METHOD FOR EVALUATING SKIN LESIONS USING ARTIFICIAL INTELLIGENCE
DISPOSITIF POUR LA MISE EN OEUVRE D'UN PROCÉDÉ POUR L'ÉVALUATION DE LÉSIONS CUTANÉES UTILISANT UNE INTELLIGENCE ARTIFICIELLE

(30) Priorität: 28.02.2019 DE 102019105152
(43) Veröffentlichungstag der Anmeldung: 05.01.2022
(73) Patentinhaber: Fotofinder Systems GmbH, 84364 Bad Birnbach (DE)
(72) Erfinder: MAYER, Andreas, 94032 Passau (DE)
(74) Vertreter: advotec.
(86) Internationale Anmeldenummer: PCT/DE2019/200121
(87) Internationale Veröffentlichungsnummer: WO 2020/173516

(56) Entgegenhaltungen:
- WO-A1-2018/160963
- WO-A1-2020/123724
- US-A1- 2014 313 303
- US-A1- 2018 061 046
- US-A1- 2018 189 949

## Beschreibung

Die Erfindung betrifft eine Vorrichtung zur Erzeugung von Abbildungen zur Unterstützung bei der Charakterisierung von Hautläsionen eines menschlichen Körpers. Insbesondere betrifft die Erfindung eine Vorrichtung zur Evaluierung von Hautläsionen bzw. Abbildungen der Hautläsionen unter Verwendung von künstlicher Intelligenz.

Ein aus dem Stand der Technik bekanntes Verfahren zur Erfassung von Hautläsionen, d.h. von Hautveränderungen und Hautschädigungen, ist die Dermatoskopie bzw. die Auflichtmikroskopie, ein nichtinvasives Untersuchungsverfahren bei dem die zu untersuchenden Hautbereiche mit einem Mikroskop unter Beleuchtung mit polarisiertem Licht bis in tiefere Hautschichten analysiert werden können. Eine Einschätzung bzw. Diagnose erfolgt hierbei durch den behandelnden Arzt mittels visueller Begutachtung der jeweiligen Hautläsion. Diese kann dann mit einer zusätzlichen histologischen Untersuchung des Hautbereichs bestätigt werden, für welche jedoch ein chirurgischer Eingriff zur Entnahme einer Gewebeprobe notwendig ist.

In der US 2018/061 046 A1 wird ein dermatoskopischer Läsionsbereich identifiziert durch: Erhalten eines dermatoskopischen Bildes und Ausführen eines Bildklassifizierers mit einem neuronalen Faltungsnetzwerk auf dem dermatoskopischen Bild, um pixelweise Läsionsvorhersagewerte zu erhalten. Segmentierung des dermatoskopischen Bildes in Superpixel und Berechnung des Durchschnitts der pixelweisen Vorhersagewerte für die Pixel innerhalb dieses Superpixels für jedes Superpixel.

Aus der US 2018/189 949 A1 ist ein Verfahren zur Überwachung von Hautanomalien auf einem Hautteil eines Patienten bekannt, das die Schritte des Empfangs erster Bilddaten von einer Bilderfassungsvorrichtung und der Identifizierung einer ersten Hautmaske, die dem Hautteil entspricht, umfasst.

Aus der US 2014/313 303 A1 ist es bekannt, dass die Entwicklung eines Hautzustandes im Laufe der Zeit bei seiner Beurteilung hilfreich sein kann. Zur Veranschaulichung: Ein Benutzer nimmt mit einem Smartphone zu verschiedenen Zeitpunkten Bilder der Haut auf. Die Bilder werden gemeinsam registriert, farbkorrigiert und dem Benutzer (oder einem Arzt) zur Überprüfung vorgelegt, z. B. in einer zeitlichen Abfolge oder als ein Bild, das als geisterhaftes Overlay über ein anderes gelegt wird. Bei der Bildregistrierung können Nävi, Haarfollikel, Falten, Poren und pigmentierte Bereiche als Schlüsselpunkte verwendet werden.

Aus der WO 2018/160 963 A1 sind Systeme und Verfahren zur nicht-invasiven klinischen Bildgebung und zur nicht-invasiven Bildgebung des subdermalen Blutflusses, die diffuse Reflexionsspektroskopie und die computergestützte Diagnose, bekannt.

Ebenso ist es bekannt, eine Aufnahme der jeweiligen Hautläsion beispielsweise mit einem an sich bekannten Videodermatoskop zu erstellen und diese für eine Evaluierung durch den jeweiligen behandelnden Arzt in entsprechenden Ausgabemitteln vergrößert und/oder wenigstens teilweise verändert, beispielsweise unter Hervorhebung spezifischer Spektralbereiche, darzustellen. Mit einer Erhöhung der Anzahl der Aufnahmen steigt jedoch der zeitliche Aufwand für eine detaillierte Begutachtung. Insbesondere bei der Evaluierung einer Vielzahl von erfassten Abbildungen unterschiedlicher oder gleicher Hautläsionen, beispielsweise im Rahmen von Nachfolgeuntersuchungen mittels derer die Veränderung einer jeweiligen Hautläsion untersucht und überwacht werden kann, ist eine zuverlässige und zeiteffiziente Charakterisierung aller Aufnahmen durch den behandelnden Arzt nicht mehr möglich.

Der vorliegenden Erfindung liegt die Aufgabe zugrunde die vorgenannten Nachteile des Standes der Technik zu überwinden oder wenigstens deutlich abzuschwächen. Insbesondere soll ein optimiertes Verfahren zur Erfassung und Unterstützung bei der Beurteilung einer Hautläsion bereitgestellt werden, welches eine effiziente und zuverlässige Erkennung von bösartigem Hautgewebe durch den behandelnden Arzt ermöglicht. Diese Aufgabe wird durch den Gegenstand des unabhängigen Anspruchs gelöst. Die Unteransprüche stellen vorteilhafte Weiterbildungen der vorliegenden Erfindung dar. Die Erfindung adressiert zudem weitere Probleme bzw. schlägt die Lösung zu weiteren Problemen vor, wie aus der nachfolgenden Beschreibung hervorgeht.

Die Erfindung betrifft eine Vorrichtung nach Anspruch 1.

Das Verfahren, zu dessen Ausführung diese Vorrichtung ausgebildet ist, aber das nicht als solches beansprucht wird, stellt eine Vorcharakterisierung einer zu untersuchenden Hautläsion bereit, welche dem behandelnden Arzt eine effiziente und sichere Analyse der jeweiligen Hautläsion ermöglicht bzw. diese für den Arzt deutlich vereinfacht. Insbesondere durch die gleichzeitige Darstellung einer Abbildung der jeweiligen Hautläsion in Kombination mit Informationen hinsichtlich einer durch das künstliche neuronale Netzwerk erkannten Klasse der Hautläsion und/oder eines zugehörigen Risikowerts wird eine effiziente und sichere Bewertung durch den behandelnden Arzt, insbesondere bei einer Vielzahl von zu untersuchenden Hautläsionen, unterstützt.

Das Verfahren wird vorzugsweise wenigstens teilweise mit einem entsprechend ausgebildeten Softwareprogramm ausgeführt. Dieses kann auf einem Speichermedium bzw. Datenträger, beispielsweise enthalten in einer unten näher beschriebenen Analyseeinheit, gespeichert sein und/oder ausgeführt werden.

Das künstliche neuronale Netzwerk ist vorzugsweise zur Erkennung vordefinierter Klassen bzw. Typen von Hautläsionen ausgebildet. Insbesondere ist das künstliche neuronale Netzwerk zur Erkennung und/oder zur Unterscheidung wenigstens zwischen nicht-melanozytären und melanozytären Hautläsionen ausgebildet. Das künstliche neuronale Netzwerk ist vorzugsweise zur Erkennung wenigstens einer Mehrzahl folgender Typen von Hautläsionen als Klassen ausgebildet: Pigmentnävus (melanozytärer Nävus), Dermatofibrom, Malignes Melanom, Aktinische Keratose und Morbus Bowen, Basalzellkarzinom (Basaliom), seborrhoische Keratose, Lentigo Solaris, Angiom, und/oder Plattenepithelkarzinom.

In einer bevorzugten Ausführungsform kann zusätzlich zur und/oder basierend auf der Analyse des künstlichen neuronalen Netzwerks eine Ausgabe bzw. Wiedergabe der erkannten Klassen bzw. Typen von Hautläsionen nach vorzugsweise abgestufter ermittelter Wahrscheinlichkeit erfolgen. Beispielsweise kann das künstliche neuronale Netzwerk nicht nur eine wahrscheinlichste Klasse bzw. einen wahrscheinlichsten Typen der Hautläsion ausgeben, sondern auch die zwei oder drei Klassen bzw. Typen mit ermittelter nachfolgender Wahrscheinlichkeit. Insbesondere können durch das künstliche neuronale Netzwerk wenigstens zwei, vorzugsweise drei oder optional auch mehr der am wahrscheinlichsten vorliegenden Klasse bzw. Typen der zu analysierenden Hautläsion ausgegeben werden. Eine derartige Ausgabe bzw. Wiedergabe kann beispielsweise sein: Basalzellkarzinom, Plattenepithelkarzinom, Angiom, wobei die einzelnen Klassen bzw. Typen vorzugsweise in absteigender Wahrscheinlichkeit ausgegeben werden. Eine derartige Ausgabe bzw. Wiedergabe ermöglicht insbesondere eine effizientere Analyse bei Hautläsionen, welche nicht klar voneinander abgrenzbar scheinen und durch die bereitgestellte Information auf effiziente Weise näher eingrenzbar sind.

In einer bevorzugten Ausführungsform ist das künstliche neuronale Netzwerk zur Erkennung von vordefinierten Risikoklassen insbesondere hinsichtlich einer Malignität der Hautläsion ausbildet. Hierbei kann eine jeweilige Risikoklasse einen jeweiligen Fortschrittsgrad einer Hautläsion wiederspiegeln. Beispielsweise kann das künstliche neuronale Netzwerk ausgebildet sein, wenigstens zwei, vorzugsweise wenigstens drei unterschiedliche Fortschrittsgrade und damit diesen zugeordnete Risikoklassen einer jeweiligen Hautläsion zu erkennen. Diese können beispielsweise als leichte, mittlere und hohe Risikoklasse unterschieden werden. Hierbei kann eine höhere Risikoklasse Fortschrittsgrade von Hautläsionen umfassen, welche als für den Menschen riskanter einzustufen bzw. welche eine zeitnahe Behandlung und/oder einen chirurgischen Eingriff benötigen. Weiter bevorzugt ist das künstliche neuronale Netzwerk ausgebildet, zwischen einer Vielzahl von unterschiedlichen Fortschrittsgraden eines Hautläsionstyps zu unterscheiden. Die jeweilige Zuordnung in entsprechende Risikoklassen kann durch das künstliche neuronale Netzwerk selbst und/oder eine der Verarbeitung durch das künstliche neuronale Netzwerk nachgelagerte Berechnung erfolgen.

In einer weiteren bevorzugten Ausführungsform kann eine jeweilige Risikoklasse auch mehrere Hautläsionstypen umfassen, welche durch das künstliche neuronale Netzwerk unterscheidbar bzw. erkennbar sind. Hierbei kann eine höhere Risikoklasse die Typen von Hautläsionen umfassen, welche als für den Menschen riskanter einzustufen sind. Dies sind insbesondere Klassen, welche eine zeitnahe Behandlung und/oder einen chirurgischen Eingriff benötigen. Weniger riskante Typen, insbesondere Typen von Hautläsionen, die keine zeitnahe Behandlung und/oder einen chirurgischen Eingriff benötigen, können als weniger riskant eingestuft und somit einer niedrigen bzw. niedrigeren Risikoklasse zugeordnet werden.

Die Einstufung eines erkannten Hautläsionstyps in eine niedrigere oder höhere Risikoklasse kann mittels des künstlichen neuronalen Netzwerks und/oder in einem weiteren Verarbeitungs- oder Berechnungsschritt des Verfahrens erfolgen. Beispielsweise können mehrere Risikoklassen und diese umfassende Hautläsionstypen in einer vordefinierten und/oder adaptierbaren Look-up Tabelle hinterlegt sein. Nach Erkennung eines bestimmten Hautläsionstyps und/oder Fortschritts des jeweiligen Typs durch das künstliche neuronale Netzwerk kann die jeweils zugehörige Risikoklasse bestimmt und/oder berechnet und anschließend ausgegeben werden.

In einer bevorzugten Ausführungsform erfolgt basierend auf einer jeweiligen erkannten oder berechneten Risikoklasse von Hautläsionstypen und/oder basierend auf einem jeweiligen erkannten Fortschrittsgrad der Hautläsion eine Ausgabe und/oder Berechnung eines vorzugsweise numerischen Risikowerts der jeweiligen Hautläsion. Der numerische Wert liegt vorzugsweise zwischen 0,1 und 1. Hierbei kann ein Wert zwischen 0,1 und 0,2 als niedriger, ein Wert zwischen 0,2 und 0,49 als mittlerer und ein Wert zwischen 0,5 und 1,0 als hoher Risikowert definiert sein. Die Berechnung des Risikowerts kann mittels des künstlichen neuronalen Netzwerks und/oder in einem weiteren Verarbeitungs- oder Berechnungsschritt des Verfahrens erfolgen.

In einer bevorzugten Ausführungsform erfolgt die Ausgabe bzw. Darstellung wenigstens einer Abbildung der erfassten Hautläsion, die Analyse der Hautläsion und/oder die Darstellung der der Abbildung zugeordneten Information in Echtzeit. Weiterhin bevorzugt ist die Abbildung der erfassten Hautläsion ein Live- bzw. Videobild der durch die Erfassungsmittel erfassten bzw. aufgezeichneten Hautläsion. Dieses kann beispielsweise mittels eines Videodermatoskops erfasst bzw. aufgenommen werden und mittels zugehöriger Ausgabemittel, beispielsweise einem Display oder Monitor einer mit den Erfassungsmitteln verbundenen Analyseeinheit, wie beispielsweise einem Computer, PC, Tablet oder Smartphone, ausgegeben werden. Durch die Bereitstellung in Echtzeit, d.h. ohne wesentliche zeitliche Verzögerung, wird neben einer vereinfachten Positionierung der Erfassungsmittel auf der jeweiligen Hautläsion eine deutlich vereinfachte Charakterisierung der Läsion durch den behandelnden Arzt aufgrund der vorzugsweise instantan bereitgestellten und zur gezeigten Läsion zugehörigen Information ermöglicht.

Die durch die Erfassungsmittel bereitgestellten Bilddaten umfassen eine Vielzahl von Einzelbildern der zu untersuchenden Hautläsion. Diese können beispielsweise im Rahmen eines kontinuierlichen Videostreams durch ein Videodermatoskop bereitgestellt werden. Die bereitgestellten Einzelbilder werden jeweils einzeln mittels des künstlichen neuronalen Netzwerks analysiert. Basierend hierauf kann dann eine jeweilige Erkennung und/oder Klassifizierung der zu untersuchenden Hautläsion durch das künstliche neuronale Netzwerk erfolgen. Die hierbei ermittelten Daten bzw. Informationen können dann zu einem Gesamtbewertungsergebnis verrechnet werden, welches dann als zur ausgegebenen Abbildung der Hautläsion zugehörig ausgegeben wird. Hierbei kann insbesondere ein Mittelwert von zuvor erfassten Einzelergebnissen bzw. Einzelklassifikationen der zu analysierenden Hautläsion gebildet und anschließend ausgegeben werden.

Das künstliche neuronale Netzwerk ist vorzugsweise ein an sich bekanntes faltendes neuronales Netzwerk, ein sogenanntes Convolutional Neural Network (CNN). Das künstliche neuronale Netzwerk weist vorzugsweise wenigstens eine verdeckte Schicht, mehr bevorzugt zwischen 1 und 100, am bevorzugtesten zwischen 1 und 20 verdeckte Schichten auf. In einer bevorzugten Ausführungsform weist das künstliche neuronale Netzwerk zwischen 2 und 10.000, vorzugsweise zwischen 2 und 1.000 Neuronen pro Schicht auf.

Das künstliche neuronale Netzwerk ist vorzugsweise ausgebildet, basierend auf mittels überwachten Lernens antrainierten Kenntnissen eine vordefinierte Klassifizierung zu erkennen. Hierbei wird dem künstlichen neuronalen Netzwerk in an sich bekannter Weise durch antrainiertes Lernen eine große Vielzahl von Hautläsionen unterschiedlichen Typs, unterschiedlicher Ausbildung und/oder unterschiedlichen Fortschritts entsprechend einem jeweiliger Diagnose vorzugsweise als Bilddaten zum Anlernen bereitgestellt. Ein derartiges Anlernen kann in an sich bekannter Weise in einem nachfolgenden Validierungsprozess hinsichtlich der Erkennungsgenauigkeit des antrainierten künstlichen neuronalen Netzwerks überprüft werden. Zudem kann mittels an sich bekannten Transferlernens ein bereits mit einem großen Datenbestand angelerntes künstliches neuronales Netzwerk verwendet werden und unter geringer Parameteränderung auf die jeweilige Verwendungsart angepasst werden. Das Anlernen und Validieren des künstlichen neuronalen Netzwerks kann beispielsweise mit Python Tensorflow und/oder Python Keras erfolgen. Die Bildverarbeitung, Bereitstellung und/oder Zuordnung kann mittels OpenCV2.4 erfolgen.

Das künstliche neuronale Netzwerk kann zudem ausgebildet sein, zuvor angelernte Kenntnisse bei der fortlaufenden Analyse der Hautläsionen aus den zugeführten Bilddaten weiter zu verbessern. Das bedeutet, dass das künstliche neuronale Netzwerk vorzugsweise selbstlernend ausgebildet ist und seine Kenntnisse während des fortlaufenden Einsatzes in der Analyse von Hautläsionen stetig erweitert bzw. verbessert. Hierbei können beispielsweise vom behandelnden Arzt bereitgestellte Informationen hinsichtlich einer jeweiligen Diagnose zu einer erfassten Hautläsion berücksichtigt werden.

Erfindungsgemäß ist die Vorrichtung dazu ausgebildet, eine Übersichtsaufnahme einer menschlichen Körperregion aufweisend eine Vielzahl von Hautläsionen, vorzugsweise ein sogenanntes "klinisches Bild", zu erfassen, und/oder eine Detailaufnahme einer Hautläsion zu einer entsprechenden Hautläsion in einer erfassten Übersichtsaufnahme vorzugsweise mittels elektronischer Datenverarbeitung automatisch zuzuordnen. Die Übersichtsaufnahme kann beispielsweise eine Ansicht bzw. Darstellung einer menschlichen Körperteilregion oder Körperregion wie beispielsweise eine menschliche Rückenansicht sein. Unter Detailaufnahme wird vorliegend eine Erfassung einer einzelnen Hautläsion, vorzugsweise aus nächster Nähe zur Hautoberfläche verstanden.

Die Erfassung der Übersichtsaufnahme erfolgt mit den Erfassungsmitteln. Diese können neben Mitteln für eine jeweilige Detailaufnahme zusätzliche Mittel, beispielsweise eine vorzugsweise hochauflösende digitale Foto- oder Videokamera zur Erfassung der Übersichtsaufnahme umfassen. Die Zuordnung einer erfassten Detailaufnahme zu einer jeweiligen Übersichtsaufnahme kann mit Hilfe eines entsprechenden Eingabemittels manuell oder mittels einer elektronischen Bildverarbeitung automatisch erfolgen. Insbesondere kann dies durch einen Vergleichsalgorithmus ermöglicht werden, welcher die jeweiligen Aufnahmen miteinander vergleicht und bei erkannter Übereinstimmung der Bilddaten eine entsprechende Zuordnung vornimmt. Hierbei kann beispielsweise eine an sich bekannte Merkmalserkennung basierend auf einer OpenCV Library verwendet werden.

Erfindungsgemäß ist die Vorrichtung dazu ausgebildet, eine neu erfasste Aufnahme, insbesondere einer Detailaufnahme, einer Hautläsion mit bereits zuvor erfassten Aufnahmen zu vergleichen. Basierend hierauf erfolgt anschließend eine Zuordnung als Wiederholungsaufnahme (Follow-up Aufnahme) zu einer bestehenden Aufnahme oder eine Neuanlage als Erstaufnahme einer Hautläsion.

Für die Zuordnung kann die jeweilige Aufnahme mit bestehenden Detailaufnahmen und einem jeweils zugehörigen Übersichtsbild verglichen werden. Beispielsweise vergleicht ein entsprechender Algorithmus eine erfasste Detailaufnahme mit bereits vorhandenen Detailaufnahmen in der Datenbank des jeweiligen Patienten. Erkennt die Bildauswertung Übereinstimmungen mit bereits bestehenden Aufnahmen, so kann diese neue Aufnahme als Follow-up Aufnahme gekennzeichnet und/oder der Läsion im Übersichtsbild als neue Aufnahme zugeordnet werden.

Erfindungsgemäß ist die Vorrichtung dazu ausgebildet, eine Analyse einer oder mehrerer Hautläsionen durch elektronische Verarbeitung einer erfassten Übersichtsaufnahme mittels des künstlichen neuronalen Netzwerks zur Erkennung und/oder Klassifizierung der jeweiligen Hautläsion durchzuführen. Insbesondere kann das künstliche neuronale Netzwerk hierbei ausgebildet sein, eine Erkennung und/oder Klassifizierung einer Vielzahl von Hautläsionen in einer erfassten Übersichtsaufnahme durchzuführen. Die Analyse der Hautläsionen in einer Übersichtsaufnahme kann hierbei parallel bzw. im Hintergrund zu einer jeweiligen Analyse einer Detailaufnahme einer Hautläsion erfolgen.

Erfindungsgemäß ist die Vorrichtung dazu ausgebildet, eine Information bei noch nicht erfolgter Erfassung einer Detailaufnahme einer jeweiligen Hautläsion einer zugeordneten Übersichtsaufnahme auszugeben, insbesondere falls basierend auf der Analyse der Übersichtsaufnahme durch das künstliche neuronale Netzwerk eine vordefinierte Klassifikation und/oder ein vordefinierter Risikowert bzw. Risikofaktor ermittelt wurde. Beispielsweise kann eine Ausgabe eines Warnhinweises oder eine graphische Hervorhebung einer Hautläsion auf einer Darstellung des Übersichtsbilds erfolgen. Basierend darauf kann der behandelnde Arzt eine Detailaufnahme der jeweiligen Hautläsion zur genaueren Beurteilung erfassen.

In einer bevorzugten Ausführungsform ist die Vorrichtung dazu ausgebildet, eine vorzugsweise regelmäßige Überprüfung der Aktualität einer jeweiligen Detailaufnahme einer Hautläsion zu einer zugeordneten Übersichtsaufnahme durchzuführen. Bei einer Überschreitung eines vordefinierten vorzugsweise absoluten Zeitwerts einer Detailaufnahme, beispielsweise für vordefinierte Monate oder Jahre, und/oder bei größeren Abweichungen von Aktualitäts- bzw. Erfassungswerten unterschiedlicher Detailaufnahmen, beispielsweise von einem jeweiligem hinterlegten Erfassungsdatum, kann dann eine Information, beispielsweise ein Warnhinweis oder eine graphische Hervorhebung einer Hautläsion auf einer Darstellung des Übersichtsbilds ausgegeben werden. Hierbei kann auf längere zurückliegende und/oder vom behandelnden Arzt nicht mehr neu angefertigte bzw. aktualisierte Detailaufnahmen hingewiesen werden. Dies kann insbesondere für Hautläsionen erfolgen, für welche basierend auf der Analyse der Übersichtsaufnahme durch das künstliche neuronale Netzwerk eine vordefinierte Klassifikation und/oder ein vordefinierter Risikofaktor ermittelt wurde.

In einer bevorzugten Ausführungsform ist das künstliche neuronale Netzwerk ausgebildet, zuvor angelernte Kenntnisse bei der Analyse der Hautläsionen in einer jeweiligen Übersichtsaufnahme aus den zugeführten Bilddaten weiter zu verbessern.

Dies erfolgt vorzugsweise fortlaufend und kann parallel bzw. im Hintergrund zu einer Analyse einer jeweiligen Detailaufnahme einer Hautläsion erfolgen. Hierbei können beispielsweise vom behandelnden Arzt bereitgestellte Informationen hinsichtlich einer jeweiligen Diagnose zu einer erfassten Hautläsion berücksichtigt werden.

In einer bevorzugten Ausführungsform werden die erfassten Aufnahmen von Hautläsionen in einer Speichereinheit, beispielsweise einer internen oder externen Speichereinheit einer Analyseeinheit gespeichert. Diese können dann mittels des künstlichen neuronalen Netzwerks im Rahmen einer vorzugsweise periodisch durchgeführten Analyse analysiert werden. Hierbei können selbst ältere Aufnahmen mit neuen Erkenntnissen des künstlichen neuronalen Netzwerks analysiert werden. Bei Abweichung von hierbei erkannten Klassifizierungen einer Hautläsion mit diesbezüglich hinterlegten Informationen oder Diagnosen, kann eine Ausgabe eines entsprechenden Hinweises erfolgen.

Die jeweiligen Abbildungen, Darstellungen und/oder Informationen können mit Ausgabemitteln beispielsweise einem Display oder Monitor einer mit den Erfassungsmitteln verbundenen Analyseeinheit, wie beispielsweise einem Computer, PC, Tablet oder Smartphone, ausgegeben werden.

Die Analyseeinheit kann beispielsweise durch einen Rechner, wie beispielsweise einen PC, Tablet oder Smartphone, gebildet sein oder diese(n) umfassen. Die Analyseeinheit umfasst vorzugsweise wenigstens eine interne oder externe Speichereinheit. Auf dieser können das künstliche neuronale Netzwerk und die für den Betrieb des Netzwerks benötigten Daten in an sich bekannter Weise gespeichert sein. Die Analyseeinheit ist zudem vorzugsweise zur Speicherung und Ausführung eines Softwareprogramms ausgebildet. Dieses kann vorzugsweise zur Durchführung des Verfahrens konfiguriert sein. Die Analyseeinheit kann zudem wenigstens eine Schnittstelle für die Anbindung der Erfassungsmittel und/oder externer oder zusätzlicher Ausgabemittel aufweisen. Die Analyseeinheit kann zudem eine Kommunikationsschnittstelle zur Anbindung an einen externen Datenserver und/oder das Internet aufweisen. Die Analyseeinheit kann weiterhin ausgebildet sein, die elektronische Verarbeitung wenigstens teilweise mit Hilfe und/oder basierend auf Informationen bereitgestellt durch externe Server und/oder Datenbankmittel durchzuführen.

Zur Vermeidung von Wiederholungen wird auf die obige Beschreibung des Verfahrens verwiesen. Insbesondere sollen die oben beschriebenen Merkmale des Verfahrens auch als für die erfindungsgemäße Vorrichtung offenbart und beanspruchbar gelten und umgekehrt.

Weitere Vorteile, Merkmale und Einzelheiten der Erfindung ergeben sich aus der nachfolgenden Beschreibung bevorzugter Ausführungsbeispiele sowie anhand der Zeichnungen, diese zeigen in:
- **Fig. 1**: eine schematische Darstellung einer bevorzugten Ausführungsform der erfindungsgemäßen Vorrichtung;
- **Fig. 2**: ein Flussdiagramm einer bevorzugten Ausführungsform des Verfahrens;
- **Fig. 3**: ein Flussdiagramm einer bevorzugten Ausführungsform der Zuordnung einer erfassten Detailaufnahme zu einer erfassten Übersichtsaufnahme;
- **Fig. 4a**: eine bevorzugte Darstellung bei der erfindungsgemäßen Ausgabe der Abbildung einer Hautläsion mit zugeordneten Informationen;
- **Fig. 4b**: eine bevorzugte Darstellung bei der erfindungsgemäßen Ausgabe der Abbildung einer Hautläsion und einer Zuordnung zu einer Übersichtsaufnahme; und
- **Fig. 4c**: eine bevorzugte Darstellung bei der erfindungsgemäßen Ausgabe einer Vielzahl von Abbildungen von erfassten Hautläsionen und den jeweils zugeordneten Informationen.

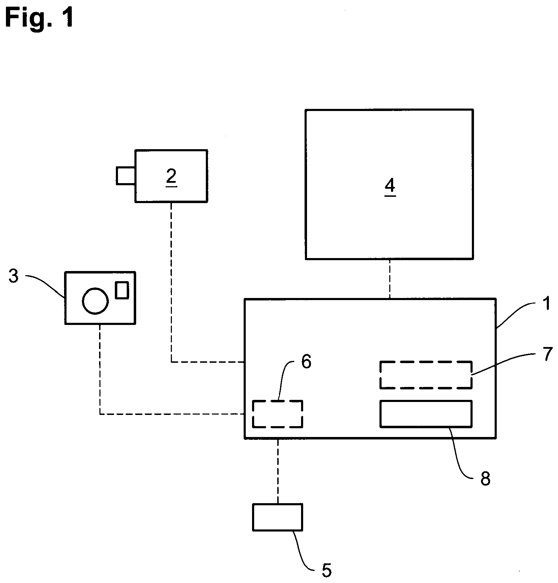

**Fig. 1** zeigt eine bevorzugte Ausführungsform einer erfindungsgemäßen Vorrichtung. Die Vorrichtung weist optische Erfassungsmittel 2 auf, welche zur Erfassung einer Aufnahme, insbesondere einer Detailaufnahme einer Hautläsion 13 eines Patienten P ausgebildet sind. Die Erfassungsmittel 2 stellen vorzugsweise digitale Bilddaten bzw. ein diese repräsentierendes Signal basierend auf der erfassten Aufnahme bereit. Die Erfassungsmittel 2 umfassen vorzugsweise ein an sich bekanntes Videodermatoskop.

Dieser kann zur Aufnahme von Detailaufnahmen einer Hautläsion im Mikroaufnahmemodus betrieben werden. Die Erfassungsmittel 2 können auch eine vorzugsweise hochauflösende digitale Bild- oder Videokamera 3 umfassen. Diese kann zur Erfassung von Detailaufnahmen und/oder einer Übersichtsaufname eines Hautbereichs eines Patienten ausgebildet sein.

Die Vorrichtung weist zudem eine Analyseeinheit 1 zur elektronischen Verarbeitung der bereitgestellten Bilddaten mittels eines künstlichen neuronalen Netzwerks auf. Die Analyseeinheit weist hierbei eine Prozessor und/oder Speichereinheit 7 auf. Auf dieser kann das künstliche neuronale Netzwerk gespeichert sein, bzw. zur Analyse der Bilddaten ausgeführt werden. Das künstliche neuronale Netzwerk ist zur Erkennung und/oder Klassifizierung von Hautläsionen ausgebildet bzw. eingerichtet. Hierbei kann das künstliche neuronale Netzwerk auf in der Speichereinheit 7 hinterlegte Daten und/oder auf einen externen Server oder eine externe Speichereinheit 5 zugreifen. Diese kann mittels einer Kommunikationsschnittstelle 6 der Analyseeinheit 1 mit der Prozessor und/oder Speichereinheit 7 verbunden sein. Die Kommunikationsschnittstelle 6 kann zudem zur Anbindung der Erfassungsmittel 2,3 an die Analyseeinheit 1 ausgebildet sein. Die Kommunikationsschnittstelle 6 kann eine drahtlose und/oder drahtgebundene Kommunikation mit den Erfassungsmitteln 2,3 und/oder dem externen Server oder einer externen Speichereinheit 5 ermöglichen.

Die Analyseeinheit 1 ist vorzugsweise ausgebildet, eine insbesondere zur Durchführung des Verfahrens geeignete Software zu umfassen bzw. bereitzustellen. Diese kann auf der Prozessor und/oder Speichereinheit 7 gespeichert und/oder ausführbar sein. Die Analyseeinheit 1 weist zudem vorzugsweise eine Benutzerschnittstelle zur Steuerung der Analyseeinheit 1 und/oder einer darauf ausgeführten Software auf. Diese kann an sich bekannte Eingabemittel wie beispielsweise eine Tastatur, Maus und/oder einen Touchscreen umfassen.

Die Vorrichtung weist zudem Ausgabemittel 4 auf, welche mit der Analyseeinheit 1 drahtlos oder drahtgebunden verbunden sind oder von der Analyseeinheit 1 umfasst sind. Die Ausgabemittel 4 sind vorzugsweise zur graphischen Wiedergabe von Informationen ausgebildet. Insbesondere können die Ausgabemittel 4 einen Bildschirm und/oder ein Touch-Display umfassen. Die Ausgabemittel 4 können zudem zur Bereitstellung von akustischen Signalen oder Warnhinweisen ausgebildet sein. Die Ausgabemittel sind insbesondere ausgebildet, eine Abbildung einer erfassten Hautläsion mit wenigstens einer zugeordneten Information basierend auf der Analyse mittels des künstlichen neuronalen Netzwerks bereitzustellen. Die Analyseeinheit 1 ist hierbei vorzugsweise ausgebildet, die Ausgabe der wenigstens einen Abbildung der erfassten Hautläsion, die Analyse der Hautläsion und/oder die Darstellung der der Abbildung zugeordneten Information in Echtzeit bereitzustellen.

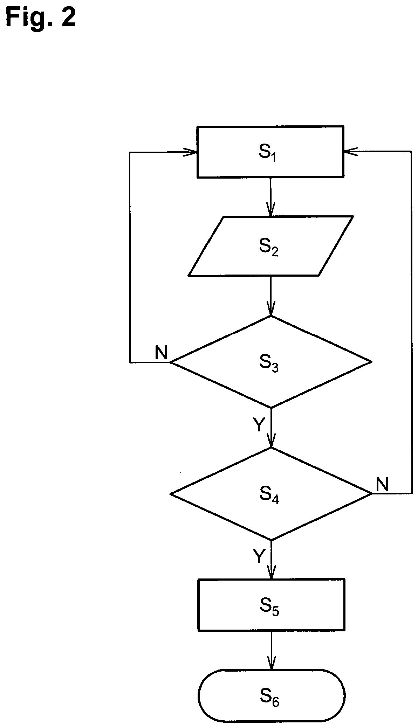

Fig. 2 zeigt ein Flussdiagramm einer bevorzugten Ausführungsform des Verfahrens. Hierbei erfolgt in einem ersten Schritt S1 die Erfassung einer Detailaufnahme einer Hautläsion mit den Erfassungsmitteln 2. Diese stellen dann ein Einzelbild oder ein Videobild aufweisend mehrere Einzelbilder bereit (S2). In einem nächsten Schritt S3 erfolgt eine Qualitätskontrolle, insbesondere, ob die Bildqualität der erfassten Aufnahme, beispielsweise hinsichtlich Bildschärfe und Ausleuchtung, ausreichend für eine Beurteilung durch das künstliche neuronale Netzwerk ist. Sollte die Bildqualität nicht ausreichend sein, beispielsweise durch eine Unschärfe des Bilds, werden die Schritte S1-S3 wiederholt. Wenn die Bildqualität ausreichend ist, erfolgt die elektronische Verarbeitung der zugehörigen Bilddaten im Rahmen der Analyse durch das künstliche neuronale Netzwerk. Diese kann einen ersten Schritt der Vorcharakterisierung S4 umfassen. In diesem wird zunächst ermittelt, ob es sich um eine Hautläsion handelt oder nicht. Falls es sich um keine Hautläsion handelt, kann eine entsprechende Ausgabe erfolgen und/oder die Schritte S1 bis S4 wiederholt werden.

In einem weiteren Schritt S5 erfolgt dann eine genaue Erkennung und/oder Klassifizierung der Hautläsion durch das künstliche neuronale Netzwerk. Hierbei kann beispielsweise eine bestimmte Klasse bzw. Typ der Hautläsion erkannt werden. Auch kann ein spezifischer Fortschritt der Hautläsion durch das künstliche neuronale Netzwerk erkannt werden. Das künstliche neuronale Netzwerk stellt dann ein der Erkennung bzw. Klassifizierung entsprechendes Ausgangssignal zur Weiterverarbeitung bereit. Des Weiteren kann das künstliche neuronale Netzwerk ausgebildet sein, basierend auf der erkannten/klassifizierten Hautläsion einen Risikofaktor zu ermitteln bzw. zu berechnen. Wenn die Hautläsion durch die Analyse als auffällig bewertet wurde, kann eine entsprechende Ausgabe an einen Benutzer erfolgen.

Die Ermittlung bzw. Berechnung des Risikofaktors kann alternativ durch einen nachgeordneten Berechnungsschritt erfolgen. Dieser kann basierend auf den bereitgestellten Daten hinsichtlich eines Typs der Hautläsion und/oder eines Fortschritts der Hautläsion eine Zuordnung zu einem vordefinierten Risikofaktor bzw. eine Einteilung in einen vordefinierten Wertebereich vornehmen. Hierfür kann ein entsprechender Softwarealgorithmus bereitgestellt sein.

In einem weiteren Schritt S6 erfolgt dann eine Ausgabe einer Abbildung der erfassten Hautläsion zusammen mit wenigstens einer weiteren Information, basierend auf den durch das künstliche neuronale Netzwerk bereitgestellten Daten. Diese kann wenigstens einen Vorcharakterisierungsparameter bzw. Klassifizierungsinformation 14 und/oder wenigstens ein ermittelter Risikofaktor 15,16 sein.

Das Verfahren zur Analyse der jeweiligen Aufnahme der Hautläsion erfolgt vorzugsweise in Echtzeit. Das Verfahren kann insbesondere durch ein entsprechendes Softwareprogramm, ausgeführt auf der zuvor beschriebenen Analyseeinheit durchgeführt werden. Die Ergebnisse der vorgenannten Schritte können einem Benutzer mit Ausgabemitteln, beispielsweise einer Anzeige- und/oder Ausgabeeinheit graphisch angezeigt werden.

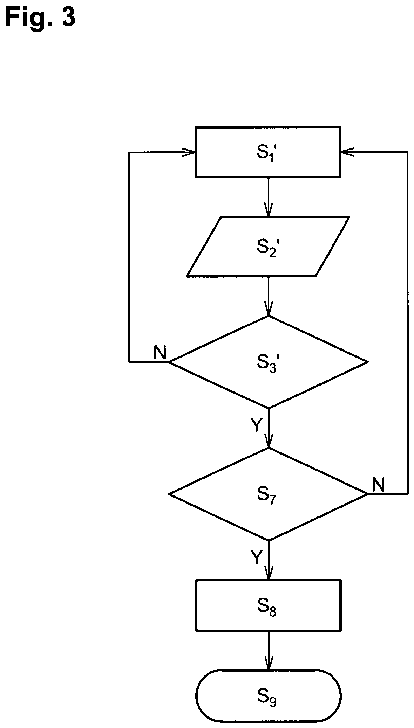

**Fig. 3** zeigt ein Flussdiagramm einer bevorzugten Ausführungsform der Zuordnung einer erfassten Detailaufnahme zu einer erfassten Übersichtsaufnahme. Eine oder mehrere Übersichtsaufnahmen können hierbei mit den Erfassungsmitteln 3 bereitgestellt werden und zeigt vorzugsweise einen größeren Haut- bzw. Körperbereich eines Patienten, wie beispielsweise einen Oberkörper in Rücken - oder Frontalansicht. Die Übersichtsaufnahme kann auch eine Ganzkörperaufnahme in Rücken- oder Frontalansicht sein. Die Übersichtsaufnahme zeigt vorzugsweise eine Vielzahl von Hautläsionen und ermöglicht eine Zuordnung von erfassten Detailaufnahmen zum Zwecke der besseren Wiederauffindbarkeit, beispielsweise bei Wiederholungsaufnahmen.

In den gezeigten Schritten S1' bis S3' erfolgt die Erfassung von Detailaufnahmen einer Hautläsion, die Bereitstellung eines entsprechenden Bild oder Videobilds und eine anschließende Qualitätskontrolle, insbesondere hinsichtlich Bildschärfe und Ausleuchtung, analog zu den vorher beschriebenen Schritten S1 bis S3. In einem anschließenden Schritt S7 erfolgt ein Abbildungsvergleich der bereitgestellten Bilddaten der Detailaufnahme mit den Bilddaten von wenigstens einem zuvor erfassten Übersichtsbild. Ein entsprechender und an sich bekannter Algorithmus vergleicht dabei die entsprechenden Bilddaten und nimmt bei einer aufgefundenen Übereinstimmung eine automatische Zuordnung des Einzelbilds zum Übersichtsbild vor. Falls keine Übereinstimmung aufgefunden wird, kann das Bild als Neuaufnahme gespeichert werden und/oder eine Erfassung eines weiteren Detailaufnahme (S1'-S3') erfolgen. Die entsprechende Zuordnung erfolgt dann in einem weiteren Schritt S8. Hierbei kann die Zuordnung direkt ausgegeben werden oder einem Benutzer als Vorschlag zur expliziten Bestätigung angezeigt werden. In einem weiteren Schritt S9 kann dann eine Anzeige des Ergebnisses der Zuordnung erfolgen. Hierbei kann beispielsweise eine Anordnung der Detailaufnahme an entsprechender Stelle in der Übersichtsaufnahme vorgenommen werden und/oder eine Markierung im Übersichtsbild und daran eine Verknüpfung zur Detailaufnahme gesetzt werden. Ein Benutzer kann dann beispielsweise mit einem Klick auf die entsprechende Stelle in einer Übersichtsaufnahme die zugeordnete Detailaufnahme öffnen.

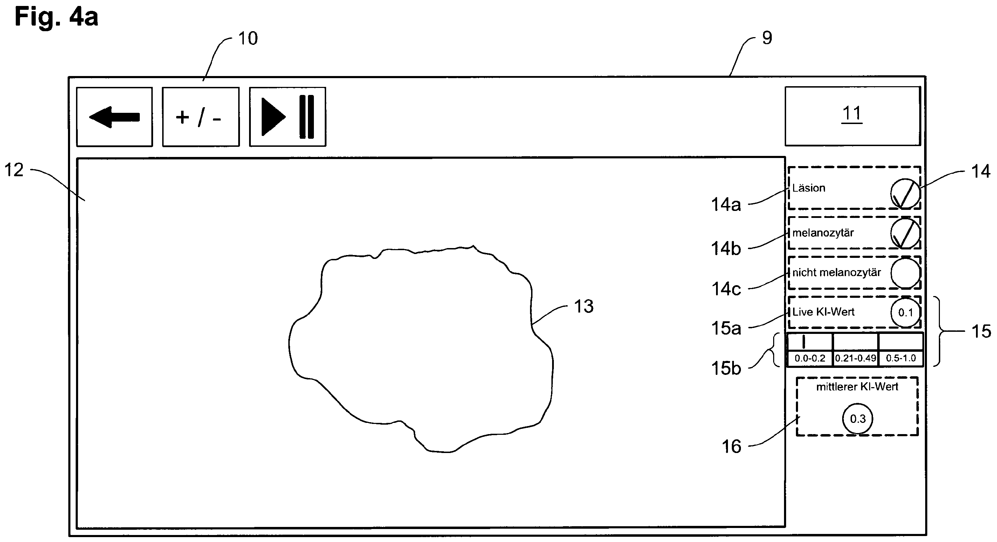

**Fig. 4a-4c** zeigen eine bevorzugte Darstellung mittels Ausgabemitteln 4 einer erfindungsgemäßen Vorrichtung bzw. entsprechende Screenshots einer graphischen Oberfläche 9 eines Softwareprogramms, welches zur Ausführung des Verfahrens ausgebildet ist.

Die Ausgabe bzw. graphische Oberfläche 9 umfasst dabei eine Abbildung 12 der erfassten Hautläsion 13. Hierbei wird vorzugsweise ein Live-Bild, d.h. eine Echtzeitabbildung einer Videoaufnahme, beispielsweise erfasst mit einem Videodermatoskop, dargestellt. Die Ausgabe bzw. graphische Oberfläche 9 umfasst vorzugsweise Navigations- und/oder Steuerungsmittel 10, mit welchen beispielsweise verschiedene Ansichten und/oder Vergrößerungsstufen der Abbildung 12 ausgewählt werden können. Ebenfalls umfasst die Ausgabe bzw. graphische Oberfläche 9 vorzugsweise Informationen zum untersuchten Patienten 11. Die Ausgabe bzw. graphische Oberfläche 9 umfasst zudem eine Information oder mehrere Informationen 14,15,16, welche zur jeweiligen abgebildeten Hautläsion 13 zugeordnet ist, und welche auf der Erkennung und/oder Klassifizierung des künstlichen neuronalen Netzwerks basieren. Die Information 14,15,16 wird hierbei vorzugsweise wenigstens teilweise in Echtzeit bereitgestellt.

Die ausgegebene Information kann insbesondere (Vor-)Klassifizierungsinformationen umfassen. Diese können einen Indikator bzw. Parameterdarstellung 14a umfassen, welche angibt, ob die analysierte Hautanomalie bzw. der erfasste Hautbereich eine Hautläsion 13 ist bzw. aufweist. Des Weiteren können die Klassifizierungsinformationen einen Indikator bzw. eine Parameterdarstellung 14b,14c umfassen, welcher angibt, ob die analysierte Hautläsion melanozytär oder nichtmelanozytär ist. Des Weiteren kann die ausgegebene Information eine jeweilig erkannte Klasse bzw. Typen der zu analysierenden Hautläsion umfassen. Beispielsweise kann ausgeben werden, ob es sich bei der Hautläsion um ein Melanom, Nävus, Basalzellkarzinom etc. handelt. Die Information kann auch zwei oder drei der mit der höchsten Wahrscheinlichkeit durch das künstliche neuronale Netzwerk erkannten Klasse bzw. Typen der zu analysierenden Hautläsion, vorzugsweise in absteigender Wahrscheinlichkeit, umfassen.

Die ausgegebene Information kann zudem eine Wiedergabe eines Risikofaktors 15 umfassen, welcher angibt, wie gesundheitsrelevant bzw. riskant die analysierte Hautläsion einzustufen ist. Dieser kann in numerischer und/oder graphischer Form 15a ausgegeben werden. Alternativ oder zusätzlich kann eine entsprechende graphische Wiedergabe in einer vordefinierten Vergleichsskala 15b erfolgen. Diese kann wenigstens in niedrig, mittel und hohes Risiko unterteilt sein. Die Ausgabe der vorgenannten Informationen erfolgt vorzugsweise in Echtzeit basierend auf der erfassten Hautläsion und der Analyse des künstlichen neuronalen Netzwerks.

Die ausgegebene Information kann zusätzlich einen Mittelwert und/oder einen durchschnittlichen Risikofaktor in numerischer und/oder graphischer Form 16 aufweisen. Dieser kann auf mehreren Einzelanalysen einer erfassten Hautläsion basieren, beispielsweise falls mehrere Einzelbilder einer spezifischen Hautläsion erfasst und analysiert werden.

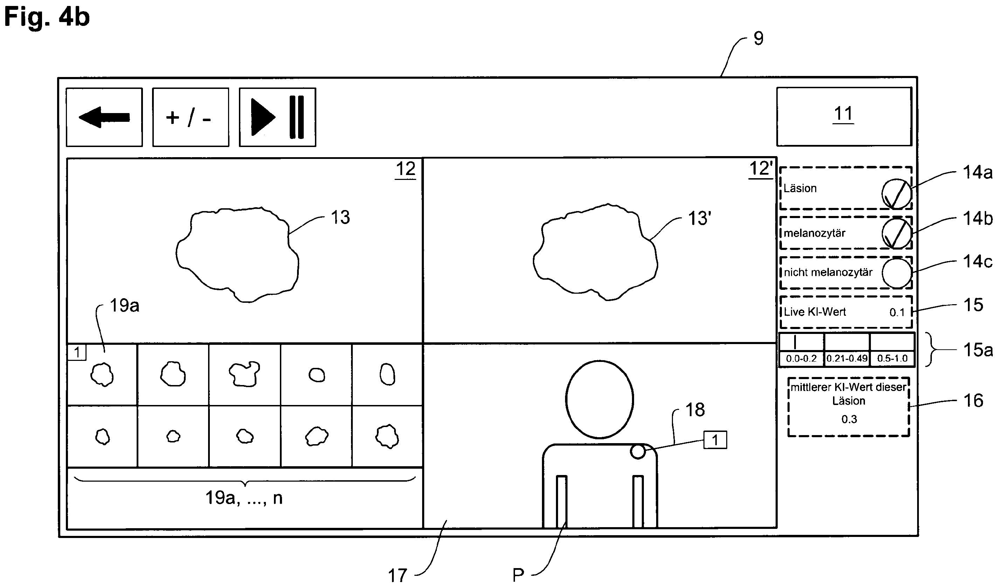

**Fig. 4b** zeigt die Zuordnung zu einer neu erfassten Detailaufnahme 12 zu einer bereits erfassten Übersichtsaufnahme 17. Hierbei wird wie zuvor mit Verweis auf Fig. 3 beschrieben, durch einen entsprechenden Bildvergleich eine automatische Zuordnung der Detailaufnahme 12 zu einer Übersichtsaufnahme 17 des Patienten P vorgenommen. Das Ergebnis der Zuordnung kann in einem separaten Fenster bzw. Bereich der graphischen Oberfläche dargestellt werden. Insbesondere kann aus einer Vielzahl von in einer Übersichtsaufnahme 17 erkannten Läsionen 19a,... 19n eine entsprechende Übereinstimmung hervorgehoben werden (19a). Zudem kann mittels eines Indikators 18 in der Übersichtsaufnahme 17 die entsprechende Position am Körper bzw. in der Übersichtsaufnahme angezeigt werden. Das Ergebnis der automatischen Zuordnung kann vorzugsweise vom Benutzer manuell angepasst werden, beispielsweise falls die automatische Zuordnung nicht korrekt ist.

Benachbart zu einer vorzugsweise Live-Darstellung der erfassten Hautläsion 13 in Abbildung 12 kann eine Referenz- bzw. die letzte für die entsprechende Läsion im Übersichtsbild erfasste Detailaufnahme 12' angezeigt werden. Die neu erfasste Detailaufnahme 12 kann hierbei als Wiederholungsaufnahme bzw. Follow-up-Aufnahme erkannt und entsprechend abgespeichert werden. Hierdurch können Bildhistoriendaten für eine jeweilige Läsion bzw. Position in einer Übersichtsaufnahme erfasst werden.

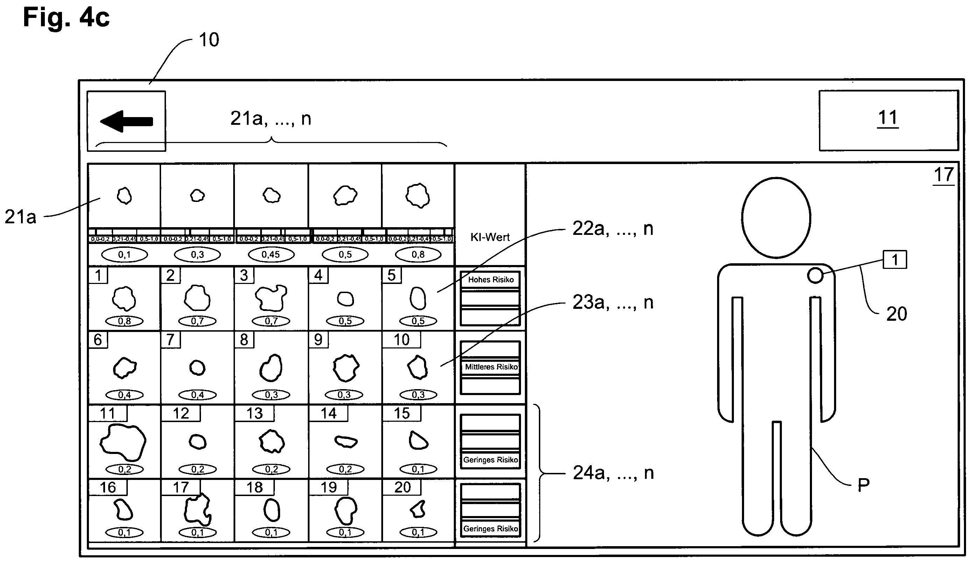

**Fig. 4c** zeigt eine bevorzugte Darstellung bei der erfindungsgemäßen Ausgabe einer Vielzahl von Abbildungen von erfassten Hautläsionen und den jeweils zugeordneten Informationen. Hierbei kann für jede der erfassten Hautläsionen eine Läsionshistorie dargestellt werden, welche aus einer Vielzahl von Detailaufnahmen 21a,...,21n besteht, die in zeitlichem Abstand, beispielsweise bei jeweiligen Untersuchungen, erfasst wurden. Für jede der Detailaufnahmen kann zudem eine zugeordnete Information wie beispielsweise ein Risikofaktor dargestellt und/oder eine Zuordnung zu einer Übersichtsaufnahme 17 mittels eines Positionsindikators 20 bereitgestellt werden. Die Darstellung kann zudem eine Unterteilung der jeweiligen Läsionen in Risikoklassen wie hoch, mittel oder niedrig umfassen. Hierbei können die die jeweilige Läsionen, für welche ein hoher Risikofaktor ermittelt wurde (22a,...2n), gesondert darstellen oder für einen Benutzer hervorgehoben werden. Die weiteren Risikoklassen wie mittel (23a,...,23n) und niedrig (24a,...,24n) können ebenfalls dargestellt sein.

Die Darstellungsweise ermöglicht es einem behandelnden Arzt die besonders riskanten Läsionen regelmäßig näher zu überprüfen. Zudem kann das künstliche neuronale Netzwerk ausgebildet sein, die jeweiligen Hautläsionen im bestehenden Bildbestand eines Patienten parallel oder im Hintergrund zu einer jeweiligen Untersuchung von Detailaufnahmen zu analysieren. Dies kann insbesondere auch basierend auf den erfassten Übersichtsaufnahmen erfolgen. Hierbei können Veränderungen von Läsionen in der Übersichtsaufnahme erkannt und/oder überprüft werden. Zudem kann eine Überprüfung erfolgen, ob der Benutzer eine jeweilige Läsion weiterhin auflichtmikroskopisch, d.h. mittels Erfassung von Detailaufnahmen, untersucht. Ist dies nicht der Fall, kann das Verfahren bzw. die Vorrichtung ausgebildet sein, mögliche Auffälligkeiten dem Benutzer aufzeigen und/oder zu empfehlen eine auffällige Läsion in die auflichtmikroskopische Untersuchung wieder mit aufzunehmen. Die oben beschriebenen Ausführungsformen sind lediglich beispielhaft, wobei die Erfindung keineswegs auf die in den Figuren gezeigten Ausführungsformen, sondern nur auf den Schutzumfang der Ansprüche beschränkt ist.

### Bezugszeichenliste

- 1: Analyseeinheit
- 2: Erfassungsmittel
- 3: Erfassungsmittel für Übersichtsaufnahme
- 4: Ausgabemittel
- 5: externer Server/Datenspeicher
- 6: Kommunikationsschnittstelle
- 7: Prozessor/Speichereinheit
- 8: Benutzerschnittstelle
- 9: Graphische Oberfläche
- 10: Navigations-/Steuerungsmittel
- 11: Patienteninformation
- 12,12': Abbildung Hautläsion (Detailaufnahme)
- 13,13': Hautläsion
- 14a-c: (Vor-)Klassifizierungsinformation
- 15a: numerische Wiedergabe Risikowert
- 15b: graphische Wiedergabe Risikowert
- 16: durchschnittlicher Risikowert
- 17: Abbildung Übersichtsbild (Übersichtsaufnahme)
- 18: Indikator automatische Zuordnung
- 19a: Darstellung zugeordnete Hautläsion
- 19b,...,n: Darstellung alternativ zuordenbarer Hautläsionen
- 20: Positionsindikator
- 21a,...,n: Läsionshistorie
- 22a,...,n: Läsionsübersicht hohes Risiko
- 23a,..., n: Läsionsübersicht mittleres Risiko
- 24a,...,n: Läsionsübersicht geringes Risiko
- P: Patient/Hautbereich
- S1,S1`: Erfassung Aufnahme
- S2,S2': Bild/Videobild
- S3,S3`: Qualitätskontrolle
- S4: Vorcharakterisierungsschritt
- S5: Erkennung und/oder Klassifizierung
- S6: Ausgabe Bewertung
- S7: Abbildungsvergleich
- S8: Abbildungszuordnung
- S9: Ausgabe Zuordnungsergebnis

## Patentansprüche

1. Vorrichtung zur Durchführung eines Verfahrens zur Darstellung wenigstens einer Abbildung einer Hautläsion und zugehöriger Information zur Unterstützung bei der Charakterisierung der Hautläsion, aufweisend
optische Erfassungsmittel (2), insbesondere ein Videodermatoskop (2), das dazu eingerichtet ist, eine Aufnahme, insbesondere einer Detailaufnahme, einer Hautläsion (13) in einem zu untersuchenden Hautbereich zu erfassen, und darauf basierende Bilddaten bereitzustellen,
eine Analyseeinheit (1) zur elektronischen Verarbeitung der bereitgestellten Bilddaten mittels eines künstlichen neuronalen Netzwerks, welches zur Erkennung und/oder Klassifizierung von Hautläsionen ausgebildet ist, und
Ausgabemittel (4), die dazu eingerichtet sind, wenigstens eine Abbildung (12) der erfassten Hautläsion (13) und eine dieser zugeordneten Information (14,15,16) basierend auf der Analyse mittels des künstlichen neuronalen Netzwerks auszugeben,
wobei die der Abbildung (12) zugeordnete Information (14,15,16) eine Wiedergabe einer erkannten vordefinierten Klasse der Hautläsion (14) und/oder eines zugehörigen numerischen Risikowerts (15,16) der Hautläsion umfasst, wobei die Bilddaten wenigstens zwei, vorzugsweise eine Vielzahl von Einzelbilder der Hautläsion (13) umfassen, wobei die Analyseeinheit (1) dazu ausgebildet ist, die Einzelbilder der Hautläsion (13) jeweils einzeln mittels des künstlichen neuronalen Netzwerks zu analysieren, und wobei die Vorrichtung dazu ausgebildet ist, zur Ausgabe der der Abbildung zugeordneten Information ein Gesamtbewertungsergebnis (16) der Einzelbilder hinsichtlich einer Erkennung und/oder Klassifizierung zu berechnen,
wobei das künstliche neuronale Netzwerk zur Erkennung von vordefinierten Risikoklassen, insbesondere hinsichtlich einer Malignität der Hautläsion (13), ausbildet ist, und/oder wobei eine Analyse der Hautläsion (13) durch die Analyseeinheit (1) eine Berechnung eines auf einer erkannten Risikoklasse der Hautläsion basierenden Risikowerts (15,16) umfasst,
wobei die Vorrichtung ferner ausgebildet ist, eine Übersichtsaufnahme (17) einer menschlichen Körperregion aufweisend eine Vielzahl von Hautläsionen zu erfassen, und/oder
eine Detailaufnahme (12) einer Hautläsion zu einer entsprechenden Hautläsion in einer erfassten Übersichtsaufnahme (17) vorzugsweise mittels elektronischer Datenverarbeitung automatisch zuzuordnen, und
wobei die Vorrichtung derart ausgebildet ist, eine neu erfasste Aufnahme einer Hautläsion (12) mit bereits zuvor erfassten Aufnahmen (12') zu vergleichen und basierend darauf eine Zuordnung als Wiederholungsaufnahme oder Neuanlage als Erstaufnahme einer Hautläsion durchzuführen,
wobei die Vorrichtung derart ausgebildet ist, eine Analyse einer oder mehrerer Hautläsionen (13) durch elektronische Verarbeitung der erfassten Übersichtsaufnahme (17) mittels des künstlichen neuronalen Netzwerks zur Erkennung und/oder Klassifizierung der jeweiligen Hautläsion durchzuführen; und
wobei die Vorrichtung derart ausgebildet ist, dass bei noch nicht erfolgter Erfassung einer Detailaufnahme einer jeweiligen Hautläsion eine Information angezeigt wird, für welche Hautläsion basierend auf der Analyse der Übersichtsaufnahme durch das künstliche neuronale Netzwerk eine vordefinierte Klassifikation und/oder ein vordefinierter Risikowert ermittelt wurde.

2. Vorrichtung nach Anspruch 1, wobei das künstliche neuronale Netzwerk zur Erkennung vordefinierter Klassen von Hautläsionen ausgebildet ist, insbesondere von nicht-melanozytären und melanozytären Hautläsionen, und/oder der Klassen Pigmentnävus, Dermatofibrom, Malignes Melanom, Aktinische Keratose und Morbus Bowen, Basalzellkarzinom (Basaliom), seborrhoische Keratose, Lentigo Solaris, Angiom, und/oder Plattenepithelkarzinom.

3. Vorrichtung nach einem der vorhergehenden Ansprüche, wobei die Ausgabe der wenigstens einen Abbildung (12) der erfassten Hautläsion (13), die Analyse der Hautläsion und/oder die Darstellung der der Abbildung zugeordneten Information (14,15,16) in Echtzeit erfolgt.

4. Vorrichtung nach einem der vorhergehenden Ansprüche, wobei das künstliche neuronale Netzwerk ausgebildet ist, basierend auf vorzugsweise mittels überwachten Lernens antrainierten Kenntnissen eine vordefinierte Klassifizierung zu erkennen und/oder wobei das künstliche neuronale Netzwerk ausgebildet ist, zuvor angelernte Kenntnisse bei der Analyse der Hautläsion aus den zugeführten Bilddaten weiter zu verbessern.

5. Vorrichtung nach einem der vorhergehenden Ansprüche, wobei das künstliche neuronale Netzwerk ein faltendes neuronales Netzwerk (CNN) ist und/oder wobei das künstliche neuronale Netzwerk wenigstens eine verdeckte Schicht aufweist.

6. Vorrichtung nach Anspruch 1, wobei die Vorrichtung derart ausgebildet ist, eine Überprüfung der Aktualität einer jeweiligen Detailaufnahme zu einer Übersichtsaufnahme (17) vorzunehmen und bei einer Überschreitung eines vordefinierten Zeitwerts und/oder bei Abweichungen von Aktualitätswerten von Detailaufnahmen (12,12') eine Information auszugeben, insbesondere für Hautläsionen, für welche basierend auf der Analyse der Übersichtsaufnahme durch das künstliche neuronale Netzwerk eine vordefinierte Klassifikation und/oder ein vordefinierter Risikowert ermittelt wurde.

7. Vorrichtung nach einem der Ansprüche 1 bis 6, wobei das künstliche neuronale Netzwerk ausgebildet ist, zuvor angelernte Kenntnisse bei der Analyse der Hautläsionen in der Übersichtsaufnahme (17) aus den zugeführten Bilddaten weiter zu verbessern.

8. Vorrichtung nach einem der vorhergehenden Ansprüche, wobei die Vorrichtung eine Speichereinheit (7) umfasst, in welcher die erfassten Aufnahmen gespeichert werden, und wobei die Vorrichtung derart ausgebildet ist, eine vorzugsweise periodische Analyse der gespeicherten Aufnahmen mittels des künstlichen neuronalen Netzwerks durchzuführen.

9. Vorrichtung nach einem der vorhergehenden Ansprüche, wobei die Analyseeinheit (1) einen Computer, PC, Tablet oder Smartphone umfasst und wobei die Ausgabemittel (4), ein Display oder Monitor umfassen.

## Claims

1. A device for carrying out a method for displaying at least one image of a skin lesion and associated information to assist in characterizing the skin lesion, the device comprising:
optical capturing means (2), in particular a video dermatoscope (2) configured to capture a picture, in particular a close-up picture, of a skin lesion (13) in an area of skin to be examined, and to provide image data based thereon,
an analyzing unit (1) for electronically processing the provided image data by means of an artificial neural network configured to identify and/or classify skin lesions, and
output means (4) configured to output at least one image (12) of the captured skin lesion (13) and information (14, 15, 16) associated with it based on the analysis by means of the artificial neural network,
wherein the information (14, 15, 16) associated with the image (12) comprises a rendition of an identified predefined class of the skin lesion (14) and/or an associated numerical risk value (15, 16) of the skin lesion, the image data comprising at least two, preferably a plurality of individual images of the skin lesion (13), the analyzing unit (1) being configured to analyze each of the individual images of the skin lesion (13) by means of the artificial neural network, and the device being configured to calculate an overall evaluation result (16) of the individual images with respect to an identification and/or classification in order to output the information associated with the image,
the artificial neural network being configured to identify predefined risk classes, in particular with respect to a malignity of the skin lesion (13), and/or an analysis of the skin lesion (13) by the analyzing unit (1) comprising calculating a risk value (15, 16) based on an identified risk class of the skin lesion,
the device being further configured to capture an overview picture (17) of a human body region comprising a plurality of skin lesions, and/or
automatically link a close-up picture (12) of a skin lesion with a corresponding skin lesion in a captured overview picture (17), preferably by electronic data processing, and
the device being configured to compare a newly captured picture of a skin lesion (12) with previously captured pictures (12') and, based thereon, link the picture as a follow-up picture or newly file the picture as a first picture of a skin lesion,
the device being configured to analyze one or more skin lesions (13) by electronically processing the captured overview picture (17) by means of the artificial neural network in order to identify and/or classify the respective skin lesion, and
if a close-up picture has not been captured yet of a respective skin lesion, the device being configured to display information indicating for which skin lesion a predefined classification and/or a predefined risk value has been determined based on the analysis of the overview picture by the artificial neural network.

2. The device according to claim 1, wherein the artificial neural network is configured to identify predefined classes of skin lesions, in particular non-melanocytic and melanocytic skin lesions, and/or the classes melanocytic nevus, dermatofibroma, malignant melanoma, actinic keratosis and Bowen's disease, basal-cell carcinoma (basalioma), seborrheic keratosis, solar lentigo, angioma, and/or squamous cell carcinoma.

3. The device according to any one of the preceding claims, wherein the outputting of the at least one image (12) of the captured skin lesion (13), the analysis of the skin lesion, and/or the displaying of the information (14, 15, 16) associated with the image takes place in real time.

4. The device according to any one of the preceding claims, wherein the artificial neural network is configured to identify a predefined classification based on knowledge preferably taught by supervised learning, and/or wherein the artificial neural network is configured to further improve previously taught knowledge while analyzing the skin lesion from the supplied image data.

5. The device according to any one of the preceding claims, wherein the artificial neural network is a convolutional neural network (CNN), and/or wherein the artificial neural network has at least one hidden layer.

6. The device according to claim 1, wherein the device is configured to check a currentness of a respective close-up picture belonging to an overview picture (17) and output information if a predefined time value has been exceeded and/or in the event of deviations from currentness values of close-up pictures (12, 12'), in particular for skin lesions for which a predefined classification and/or a predefined risk value has been determined based on the analysis of the overview picture by the artificial neural network.

7. The device according to any one of claims 1 to 6, wherein the artificial neural network is configured to further improve previously taught knowledge during the analysis of the skin lesions in the overview picture (17) from the supplied image data.

8. The device according to any one of the preceding claims, wherein the device comprises a memory unit (7) in which the captured pictures are stored, and the device is configured to analyze the stored pictures, preferably periodically, by means of the artificial neural network.

9. The device according to any one of the preceding claims, wherein the analyzing unit (1) comprises a computer, a PC, a tablet or a smartphone, and the output means (4) comprise a display or a monitor.

## Revendications

1. Dispositif pour la mise en œuvre d'un procédé d'affichage d'au moins une image d'une lésion cutanée et d'informations associées afin d'aider à caractériser la lésion cutanée, le dispositif comprenant :
des moyens de capture (2) optiques, notamment un dermatoscope vidéo (2) configuré pour capturer une prise de vue, notamment une prise de vue détaillée, d'une lésion cutanée (13) dans une zone de peau à examiner et pour fournir des données d'image sur la base de celle-ci,
une unité d'analyse (1) pour traiter électroniquement les données d'image fournies au moyen d'un réseau de neurones artificiels configuré pour identifier et/ou classer des lésions cutanées et
des moyens de sortie (4) configurés pour fournir en sortie au moins une image (12) de la lésion cutanée (13) capturée et une information (14, 15, 16) associée à celle-ci sur la base de l'analyse au moyen du réseau de neurones artificiels,
dans lequel l'information (14, 15, 16) associée à l'image (12) comprend une représentation d'une classe prédéfinie identifiée de la lésion cutanée (14) et/ou une valeur de risque (15, 16) numérique associée de la lésion cutanée, les données d'image comprenant au moins deux images individuelles, de préférence une pluralité d'images individuelles, de la lésion cutanée (13), l'unité d'analyse (1) étant configurée pour analyser chacune des images individuelles de la lésion cutanée (13) au moyen du réseau de neurones artificiels et le dispositif étant configuré pour calculer un résultat d'évaluation global (16) des images individuelles en vue d'une identification et/ou une classification afin de fournir en sortie l'information associée à l'image,
le réseau de neurones artificiels étant configuré pour identifier des classes de risque prédéfinies, notamment en ce qui concerne une malignité de la lésion cutanée (13), et/ou une analyse de la lésion cutanée (13) par l'unité d'analyse (1) comprenant le calcul d'une valeur de risque (15, 16) basée sur une classe de risque identifiée de la lésion cutanée,
le dispositif étant configuré en outre pour capturer une prise de vue d'ensemble (17) d'une région de corps humaine présentant une pluralité de lésions cutanées et/ou
pour attribuer une prise de vue détaillée (12) d'une lésion cutanée à une lésion cutanée correspondante dans une prise de vue d'ensemble (17) capturée, de préférence par traitement électronique de données, et
le dispositif étant configuré pour comparer une prise de vue nouvellement capturée d'une lésion cutanée (12) à des prises de vue (12') capturées précédemment et, sur cette base, effectuer une attribution comme prise de vue répétée ou effectuer une nouvelle création comme première prise de vue d'une lésion cutanée,
le dispositif étant configuré pour analyser une ou plusieurs lésions cutanées (13) en traitant électroniquement la prise de vue d'ensemble (17) capturée au moyen du réseau de neurones artificiels afin d'identifier et/ou de classer la lésion cutanée en question et
le dispositif étant configuré de telle sorte que, si aucune prise de vue détaillée d'une lésion cutanée en question n'a encore été capturée, une information est affichée indiquant pour quelle lésion cutanée une classification prédéfinie et/ou une valeur de risque prédéfinie a été déterminée sur la base de l'analyse de la prise de vue d'ensemble par le réseau de neurones artificiels.

2. Dispositif selon la revendication 1, dans lequel le réseau de neurones artificiels est configuré pour identifier des classes prédéfinies de lésions cutanées, notamment de lésions cutanées non-mélanocytaires et mélanocytaires, et/ou les classes naevus mélanocytaire, dermatofibrome, mélanome malin, kératose actinique et maladie de Bowen, carcinome basocellulaire (basaliome), kératose séborrhéique, lentigo solaire, angiome et/ou carcinome épidermoïde.

3. Dispositif selon l'une quelconque des revendications précédentes, dans lequel la fourniture en sortie de l'au moins une image (12) de la lésion cutanée (13) capturée, l'analyse de la lésion cutanée et/ou l'affichage de l'information (14, 15, 16) associée à l'image s'effectuent en temps réel.

4. Dispositif selon l'une quelconque des revendications précédentes, dans lequel le réseau de neurones artificiels est configuré pour identifier une classification prédéfinie sur la base de connaissances acquises de préférence par apprentissage supervisé et/ou dans lequel le réseau de neurones artificiels est configuré pour améliorer encore des connaissances acquises précédemment lors de l'analyse de la lésion cutanée à partir des données d'image fournies.

5. Dispositif selon l'une quelconque des revendications précédentes, dans lequel le réseau de neurones artificiels est un réseau neuronal convolutif (CNN) et/ou dans lequel le réseau de neurones artificiels comporte au moins une couche cachée.

6. Dispositif selon la revendication 1, dans lequel le dispositif est configuré pour vérifier l'actualité d'une prise de vue détaillée en question appartenant à une prise de vue d'ensemble (17) et pour fournir en sortie une information en cas de dépassement d'une valeur temporelle prédéfinie et/ou en cas d'écarts par rapport à des valeurs d'actualité de prises de vue détaillées (12, 12'), notamment pour des lésions cutanées pour lesquelles une classification prédéfinie et/ou une valeur de risque prédéfinie a été déterminée sur la base de l'analyse de la prise de vue d'ensemble par le réseau de neurones artificiels.

7. Dispositif selon l'une quelconque des revendications 1 à 6, dans lequel le réseau de neurones artificiels est configuré pour améliorer encore des connaissances acquises précédemment lors de l'analyse des lésions cutanées dans la prise de vue d'ensemble (17) à partir des données d'image fournies.

8. Dispositif selon l'une quelconque des revendications précédentes, dans lequel le dispositif comprend une unité de mémoire (7) dans laquelle les prises de vue capturées sont stockées et dans lequel le dispositif est configuré pour analyser de préférence périodiquement les prises de vue stockées au moyen du réseau de neurones artificiels.

9. Dispositif selon l'une quelconque des revendications précédentes, dans lequel l'unité d'analyse (1) comprend un ordinateur, un PC, une tablette ou un smartphone et dans lequel les moyens de sortie (4) comprennent un écran ou un moniteur.
